(19) **Europäisches Patentamt / European Patent Office / Office européen des brevets**

(11) **EP 3 831 936 A1**

(12) # EUROPEAN PATENT APPLICATION
published in accordance with Art. 153(4) EPC

(43) Date of publication:
**09.06.2021 Bulletin 2021/23**

(21) Application number: **19844833.4**

(22) Date of filing: **30.07.2019**

(51) Int Cl.:
*C12N 5/0783* (2010.01)　　*A61K 35/17* (2015.01)
*A61P 35/00* (2006.01)　　*C12N 5/10* (2006.01)
*A61K 35/545* (2015.01)

(86) International application number:
**PCT/JP2019/029750**

(87) International publication number:
**WO 2020/027094 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **31.07.2018 JP 2018144514**

(71) Applicant: **Thyas Co. Ltd.**
**Kyoto-shi, Kyoto 606-8304 (JP)**

(72) Inventors:
• **YASUI Yutaka**
**Kyoto-shi Kyoto 606-8304 (JP)**

• **HITOSHI Yasumichi**
**Kyoto-shi Kyoto 606-8304 (JP)**
• **UENO Hiroyuki**
**Kyoto-shi Kyoto 606-8304 (JP)**
• **KATSURA Yoshimoto**
**Kyoto-shi Kyoto 606-8304 (JP)**
• **KANEKO, Shin**
**Kyoto-shi Kyoto 606-8501 (JP)**

(74) Representative: **Lederer & Keller Patentanwälte Partnerschaft mbB**
**Unsöldstraße 2**
**80538 München (DE)**

(54) **METHOD FOR PRODUCING REGENERATED T CELL POPULATION VIA IPS CELLS**

(57)　The present invention provides a method for producing a regenerated T cell population via iPS cells, wherein the said regenerated T cell population maintains the repertoire diversity of a T cell population before initialization and has specificity to an antigen. The method comprises steps of (1) bringing a T cell population into contact with one or more tumor-related antigens, or a biological tissue containing one or more tumor-related antigens, or a crushed material or a lysate thereof, and enriching the T cell population having specificity to the tumor-related antigens, (2) initializing the enriched T cell population to iPS cells, and culturing while maintaining the repertoire diversity of the enriched T cell population, and (3) producing a regenerated T cell population from the cultured iPS cells.

Figure 2

EP 3 831 936 A1

## Description

### TECHNICAL FIELD

The present invention relates to a method for producing regenerated T cell population having repertoire diversity, a regenerated T cell population produced by the method, and a pharmaceutical composition containing the regenerated T cell population.

### BACKGROUND ART

T cells play a central role in immune response to abnormal cells such as foreign pathogens, like bacteria or viruses, or cancer cells. Accordingly, lowering of the function of T cells is considered to contribute to the generation of pathogen infection and cancer. For diseases caused by lowering of the function of T cells, the replenishment or regeneration of T cells can be an extremely effective means for ameliorating or treating the diseases.

T cell replenishment therapy using T cells induced from pluripotent stem cells such as iPS cells has been proposed. It is possible to produce regenerated T cells exhibiting the same antigen specificity as original T cells by using T cells specific to target antigens as raw materials for producing the iPS cells (non-patent document 1, The contents of this document are incorporated herein by reference in its entirety.). According to this method, established iPS cells are initialized in a cell population (colony) unit derived from single cells. By using the method, it is possible to use an iPS cell line which is confirmed by means such as gene analysis to be stable in cell properties, to have good differentiation efficiency to target cell or tissue, and to have no mutation or abnormality. In order to confirm the quality of clones and their compatibility with patients, it is necessary to inspect clones one by one. Therefore, the cloning is considered to be an important process.

On the other hand, it has been known from studies using human and mouse subjects that when T cell replenishment therapy is performed to infectious diseases and tumors by using a T cell population having repertoire diversity, high therapeutic effect can be achieved, because it is possible to induce a polygonal immune response to a target tissue or antigen, and it is hardly affected by lowering of the expression of antigen or immunity escape by mutation (immune sequence).

As prior art, gene-modified T cells into which a gene of a T cell receptor (TCR) or a chimeric antigen receptor (CAR) is introduced has been reported. However, the gene sequence of the receptor to be introduced is single (patent documents 1 to 3). In addition, a method for producing T cells having the same antigen specificity as the original T cells by re-differentiating iPS cells produced from peripheral blood T cells has been reported. However, their repertoire diversity is not indicated (patent document 4).

### PRIOR ART LITERATURE

#### Patent Documents

1. Japanese patent publication No. 2017-534261
2. Japanese patent publication No. 2017-530694
3. Japanese patent publication No. 2018-514204
4. Japanese patent publication No. 2017-158559

#### Non-patent Document

1. Nishimura T, et al. Generation of rejuvenated antigen-specific T cells by reprogramming to pluripotency and re-differentiation. Cell Stem Cell. 2013 Jan 3; 12(1): 114-126.

### SUMMARY OF THE INVENTION

#### Problems to be Solved by the Invention

Usually, in a method utilizing an iPS cell line, cloning is performed. The cloning is meant to remove clones except the selected clone. According, when the iPS cells established from T cells are cloned, the repertoire diversity present in the original T cell population is completely lost. This means that only an immune reaction targeting a single antigen epitope can be used. However, in an immune reaction targeting a single antigen epitope, there are problems in that it is easy to be affected by the lowering of the expression of the antigen molecule or the immune escape by mutation (immune sequence), and it is difficult to achieve high therapeutic effect in T cell replenishment therapy. On the other hand, in a T cell replenishment therapy which is conventionally performed, a T cell population having repertoire diversity can be used. However, there are problems in that T cells are exhausted by amplification of a T cell population outside the body, and immune response to antigen is lowered. Therefore, it is desired to develop a new method for producing a regenerated T cell population having repertoire diversity for use in T cell replenishment therapy.

The purpose of the present invention is to provide a method for producing a regenerated T cell population having repertoire diversity via iPS cells, and a regenerated T cell population produced by the method. The present invention targets on solving the problems of single antigen epitope and exhaustion of T cell population by providing the regenerated T cell population. Moreover, the present invention targets on providing a pharmaceutical composition containing the regenerated T cell population and a cancer treatment method using the pharmaceutical composition.

**Means for Solving the Problems**

The purpose of the present invention is achieved by the following inventions.

[1] A method for producing a regenerated T cell population via iPS cells, comprising the steps of:

1. bringing a T cell population into contact with one or more tumor-related antigens, or a biological tissue containing one or more tumor-related antigens, or a crushed material or a lysate thereof, and enriching the T cell population having specificity to the tumor-related antigens,
2. initializing the enriched T cell population to iPS cells, and culturing while maintaining the repertoire diversity of the enriched T cell population, and
3. producing a regenerated T cell population from the cultured iPS cells.

[2] A method according to [1], wherein the regenerated T cell population maintains the repertoire diversity of the enriched T cell population and has specificity to the tumor-related antigens.
[3] A method according to [1] or [2], wherein the T cell population in step 1 is derived from a mammal.
[4] A method according to [3], wherein the mammal is a human.
[5] A method according to [4], wherein the human is a cancer patient or a non-cancer patient.
[6] A method according to [5], wherein the cancer patient or the non-cancer patient has been administered, or currently being administered, or scheduled to be administered cancer vaccine.
[7] A method according to any of [1] to [6], wherein the T cell population in step 1 is derived from body fluid.
[8] A method according to [7], wherein the body fluid is selected from the group consisting of blood, lymph fluid, tissue fluid, body cavity fluid (including ascites, pleural fluid, pericardial fluid, cerebrospinal fluid, joint fluid and aqueous humor), nasal juice and urine.
[9] A method according to any of [1] to [6], wherein the T cell population in step 1 is T cells derived from a tumor tissue.
[10] A method according to any of [1] to [9], wherein the T cell population in step 1 is $\alpha\beta$T cells, $\gamma\delta$T cells, helper T cells, regulatory T cells, cytotoxic T cells, natural killer T cells, or a mixture thereof.
[11] A method according to any of [1] to [10], wherein the biological tissue in step 1 is a cancer primary tissue and a cancer metastasis tissue.
[12] A method according to any of [1] to [11], wherein the step of enriching the T cell population in step 1 comprises steps of activating by contacting with one or more tumor-related antigens, and separating the proliferated T cells.
[13] A method according to any of [1] to [12], wherein the step of enriching the T cell population in step 1 comprises one or more steps selected from the group consisting of a step of selecting CD137 expression T cells and a step of selecting IFN-$\gamma$ producing T cells from the T cell population.
[14] A method according to any of [1] to [12], wherein the step of enriching the T cell population in step 1 comprises one or more steps selected from the group consisting of a step of selecting CD137 expression T cells, a step of selecting IFN-$\gamma$ producing T cells, and a step of selecting T cells to which the antigen peptide-HLA complex binds from the T cell population.
[15] A method according to any of [1] to [14], wherein the step 2 comprises recovering iPS cells without cloning and passaging.
[16] A method according to any of [1] to [15], wherein the regenerated T cell population obtained in step 3 is used for T cell replenishment therapy.
[17] A method according to any of [1] to [16], wherein the type of V$\beta$ to be maintained by the regenerated T cell population is two or more.
[18] A regenerated T cell population produced by the method according to any of [1] to [17].
[19] A regenerated T cell population according to [18] which maintains repertoire diversity of T cell population present in living body.

[20] A pharmaceutical composition which contains the regenerated T cell population according to [18] or [19].

[21] A pharmaceutical composition according to [20] for treating a cancer treatment subject by autologous or allogeneic transplantation.

[22] A method for treating cancer which uses the pharmaceutical composition according to [20] or [21].

**EFFECTS OF THE INVENTION**

According to the present invention, it becomes possible to produce a regenerated T cell population having specificity to an antigen and repertoire diversity via iPS cells. By stimulating a T cell population present in a living body with a tumor-related antigen, T cells having specificity to the tumor-related antigen and repertoire diversity can be enriched. However, by initializing the enriched T cell population to iPS cells, and re-differentiating into a T cell population, a regenerated T cell population maintaining specificity to the tumor-related antigen of the enriched T cell population and repertoire diversity can be obtained. The regenerated T cell population thus obtained can exhibit high therapeutic effect in T cell replenishment therapy. In particular, tumor infiltrating lymphocytes (TIL) T cells are known to be specific to tumors and recognize a variety of antigens. By producing a regenerated TIL population from TIL via initialization to iPS cells, tumor-specific and effective T cell replenishment therapy by T cells that have repertoire diversity and have been rejuvenated can be performed.

**BRIEF DESCRIPTION OF THE DRAWINGS**

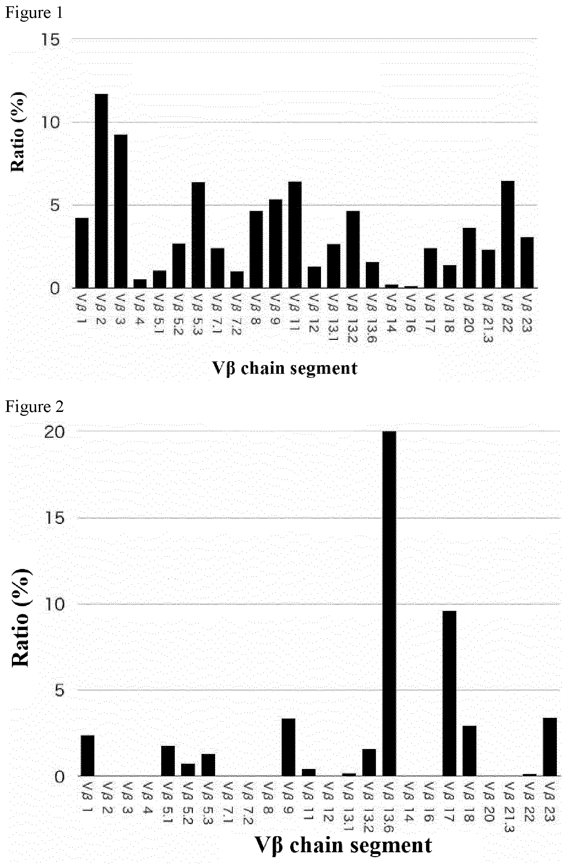

FIG. 1 is a diagram showing that a T cell population separated from an extirpated tumor of a patient with lung cancer has diversity in TCR β chains. The Vβ1-Vβ23 of the horizontal axis show the type of the TCR β chains (24 types). The vertical axis shows the proportion of the T cells having respective TCR β chains. For each type of the Vβ, although the contained proportion is different (about 1-12%), all Vβ is present in the T cell population. However, since there is undetected Vβ due to detection limit of the TCR repertoire kit used in this example, the total proportion of the present figure is not 100%.

FIG. 2 is a diagram showing that a regenerated T cell population produced via iPS cells from a T cell population separated from an extirpated tumor of a patient with lung cancer has diversity in TCR β chains. 24 types of Vβ in total existed in the original T cell population (FIG. 1). Meanwhile, there are 13 types of Vβ (Vβ1, Vβ5.1, Yβ5.2, Vβ5.3, Vβ9, Vβ11, Vβ13.1, Vβ13.2, Vβ13.6, Vβ17, Vβ18, Vβ22 and Vβ23) contained in the regenerated T cell population. About 54% of the diversity of Vβ is preserved.

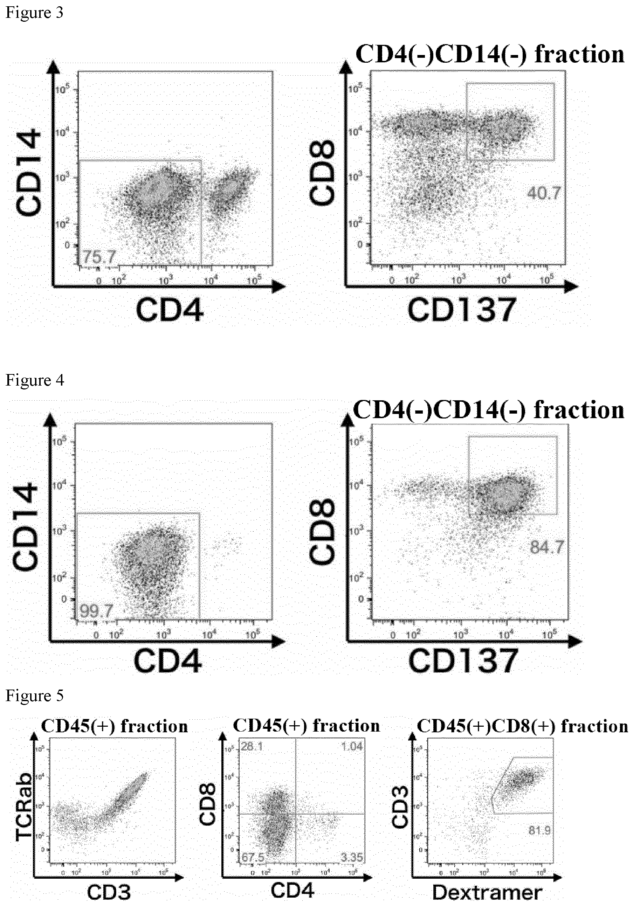

FIG. 3 is a diagram showing a result obtained by performing amplification culture using GPC3 peptide and analyzing a T cell population expressing an activation marker by a flow cytometer. A fraction which did not express any of CD4 and CD14 (left figure) was gated and developed with CD8 and CD137 (right figure). It is indicated by the figure that CD8/CD137 double-positive cells having specificity to GPC3 were amplified at a high rate.

FIG. 4 is a diagram showing a result obtained by performing amplification culture using GPC3 peptide, enriching a cell population expressing both CD8 and CD137 for a T cell population expressing activation marker, and analyzing by a flow cytometer in the same manner as in FIG. 3. It is indicated that the cells expressing the CD4 have been removed by the CD8 negative selection (left figure) when compared to FIG. 3. Besides, it is indicated that cells not expressing CD137 have also been removed by the CD137 positive selection (right figure). Therefore, CD8/CD137 double-positive cells having specificity to GPC3 are enriched at a high rate.

FIG. 5 is a diagram showing a result of re-differentiation induction of iPS cells initialized from a T cell population having specificity to GPC3 into CD8 single-positive T cells. The cells with which the re-differentiation induction was performed were stained with an antibody (CD3-APC/Cy7, CD4-BV421, CD8-PerCP/Cy5.5, TCRab-FITC, CD45-BV510, and GPC3-HLA complex (Dextramer)-APC). The ratio of the T cells having specificity to GPC3 was analyzed by using a flow cytometer (BD FACSAria® II).

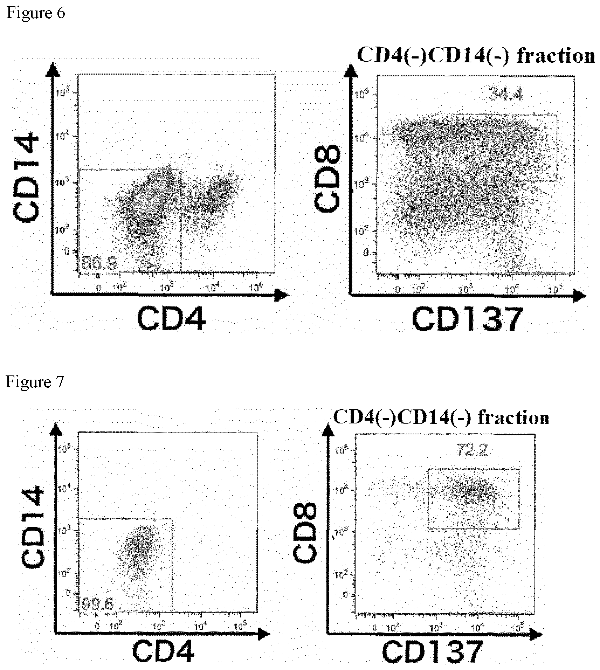

FIG. 6 is a diagram showing the results obtained by performing amplification culture of peripheral blood mononuclear cells of a non-cancer patient (healthy human subject) by using WT1 overwrap peptide, and analyzing the T cell population expressing activation marker in the same manner as in FIG. 3. It is indicated by the figure that CD8/CD137 double-positive cells having specificity to WT1 are amplified at a high rate.

FIG. 7 is a diagram showing the results obtained by performing amplification culture of a T cell population expressing activation marker by using WT1 overwrap peptide, and analyzing in the same manner as in FIG. 4. It is indicated by the figure that CD8/CD137 double-positive cells having specificity to WT1 are enriched at a high rate.

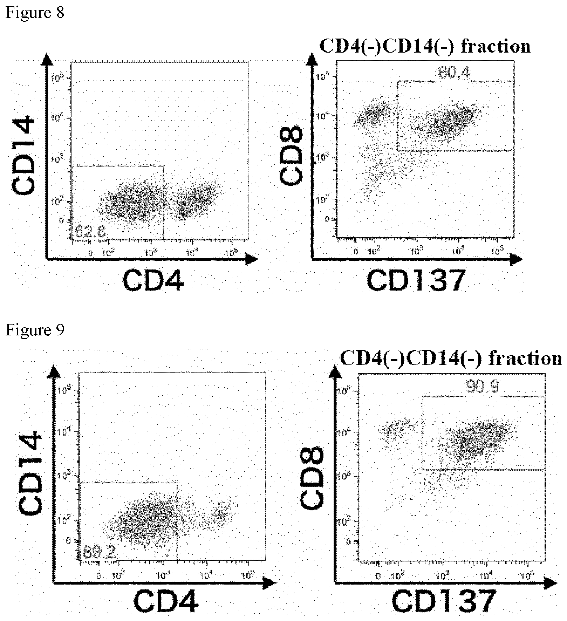

FIG. 8 is a diagram showing the results obtained by performing amplification culture of peripheral blood mononuclear cells of a non-cancer patient (healthy human subject) by using EBV LMP2A overwrap peptide, and analyzing the T cell population expressing activation marker in the same manner as in FIG. 3. It is indicated by the figure that CD8/CD137 double-positive cells having specificity to EBV LMP2A are amplified at a high rate.

FIG. 9 is a diagram showing the results obtained by performing amplification culture of a T-cell population expressing activation marker by using EBV LMP2A overwrap peptide, and analyzing in the same manner as in FIG. 4. It is indicated by the figure that CD8/CD137 double-positive cells having specificity to EBV LMP2A are enriched at a high rate.

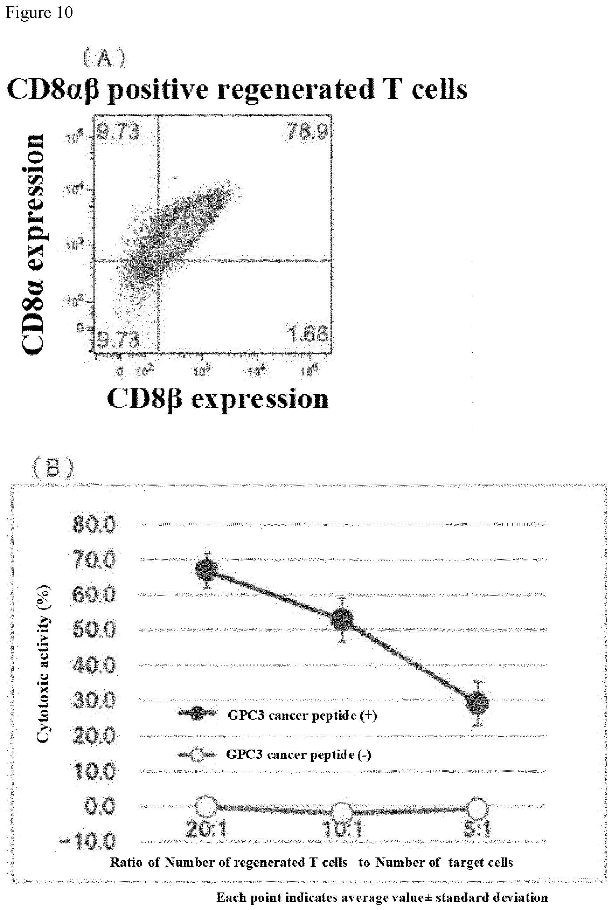

FIG. 10 is a diagram showing that a regenerated T cell population obtained by re-differentiation-inducing iPS cells initialized from a T cell population having specificity to GPC3 to CD8 single-positive T cells has a specific cytotoxic activity to target cells expressing GPC3 peptide. (A) is a diagram showing a result of flow cytometry analysis of a regenerated T cell population. (B) is a diagram showing a result of comparing the cytotoxic activity of the regenerated T cell population by target cells expressing or not expressing GPC3 peptide.

## EMBODIMENTS FOR CARRYING OUT THE INVENTION

The present invention provides a method for producing a regenerated T cell population via iPS cells. The method comprises step 1 of bringing a T cell population into contact with one or more tumor-related antigens, or a biological tissue containing one or more tumor-related antigens, or a crushed material or a lysate thereof, and enriching the T cell population having specificity to the tumor-related antigens, step 2 of initializing the enriched T cell population to iPS cells and culturing while maintaining the repertoire diversity of the enriched T cell population, and step 3 of producing a regenerated T cell population from the cultured iPS cells. Furthermore, the regenerated T cell population obtained by the present invention is characterized by maintaining the repertoire diversity of the enriched T cell population and having specificity to the tumor-related antigen.

In the present invention, "a method for producing a regenerated T cell population via iPS cells" refers to a method for regenerating a T cell population having specificity to an antigen while maintaining repertoire diversity from a T cell population having repertoire diversity via initialization to iPS cells. In the present invention, a T cell population produced by the method is referred to as a "regenerated T cell population". The "T cell" refers to a cell expressing an antigen receptor called a T cell receptor (TCR) on cell surface.

[STEP 1]

In step 1 of the present invention, a T cell population having repertoire diversity is brought into contact with one or more tumor-related antigens, or a biological tissue containing one or more tumor-related antigens, or a crushed material or a lysate thereof. The T cell population having specificity to the tumor-related antigens is enriched.

In the present invention, a "T cell population" is a lymphocyte that is positive for CD3 and CD45. It is variety of cell population where gene sequence of T cell receptors (TCR) that recognize antigens are as populations. Thus, T cells collected from a living body are T cell populations having specificity for various antigens. T cells present in a living body have a TCR gene sequence different in individual T cells by generating random recombination of the TCR gene when T progenitor cells are generated and differentiated in the thymus, and can cause an immune response to any antigen. Although the antigen or peptide sequence specifically recognizing the TCR possessed by individual T cells is determined, it can be understood by considering T cells as a population that their T cell populations take various antigens as immune targets. Thus, the T cell population collected from a living body is a T cell population having repertoire diversity in this sense. The iPS cells established from such T cell populations do not express the TCR gene. However, gene information of the TCR generated by recombination is maintained in the DNA of the iPS cells. iPS cells maintaining repertoire diversity are iPS cells established from T cell populations having repertoire diversity. Individual cells are referred to cell populations having different gene sequences of TCR.

In the present invention, T cells are preferably derived from a mammal, and more preferably derived from a human subject (a cancer patient or a non-cancer patient). Preferably, the cancer patient or non-cancer patient has been administered, is currently administered, or is scheduled to be administered a cancer vaccine. One or more cancer vaccines may be administered. Examples of T cells include, but are not limited thereto, $\alpha\beta$T cells, $\gamma\delta$T cells, helper T cells, regulatory T cells, cytotoxic T cells, and natural killer (NK) T cells. In addition, source for collecting T cells is preferably, but not limited thereto, peripheral blood because of its low invasiveness. Other preferable sources for collection include all sources in vivo, such as cancer tissue or tumor tissue or other tissue or organ, or blood, umbilical cord blood, lymph fluid, tissue fluid (interstitial fluid, intercellular fluid and interstitial fluid), body cavity fluid (ascites, pleural fluid, pericardial fluid, cerebrospinal fluid, joint fluid and aqueous humor), nasal juice, urine, and the like. In one embodiment of the present invention, T cells are preferably derived from a tumor tissue. The T cells derived from a tumor tissue are usually tumor infiltration T cells.

A "cancer vaccine" is a composition containing a vaccine antigen for inducing immune response specific for cancer or tumor, derived from cancer or tumor-related antigens, which is a cancer or tumor-specific protein or peptide.

A cell vaccine such as dendritic cells antigen-pulsed with a cancer tissue, protein or peptide that is an antigen specific to cancer or tumor, a viral DNA or RNA vaccine that expresses an antigen in an administered living body, and a composition containing cancer or tumor cells in which proliferation is suppressed and a crushed material or a lysate thereof are also included in the "cancer vaccine". Furthermore, a non-specific immunopotentiator such as a bacterial cell, a bacterial extract or a β-glucan, and a tumor-soluble virus such as adenovirus are also included in the "cancer vaccine". Typically, a cancer vaccine contains adjuvant for enhancing a specific immune response induced by vaccine antigen. After being administered to a living body, a vaccine antigen contained in a cancer vaccine is phagocytosed by antigen presenting cells (mainly dendritic cells and macrophages), treated inside the cells, and turned into an epitope peptide comprising an amino acid of about 8-30 residues, and presented on the surface of the antigen presenting cell as a complex combined with major histocompatibility complex (MHC) class I or class II. The length of the epitope peptide is 8-11 residues of amino acids in case of MHC class I, and 13-30 residues of amino acids in case of MHC class II. The T cell receptor (TCR) expressing on the surface of cytotoxic T cells and helper T cells specifically recognizes MHC class I/peptide complex or MHC class II/peptide complex, respectively. As a result, these T cells are activated to exert an antitumor effect. That is, the cytotoxic T cells recognize and destroy cancer cells presenting the same epitope peptide as that contained in the vaccine antigen. The helper T cells enhance the action of cytotoxic T cells through secretion of cytokines and chemokines such as interferon (IFN)-γ and interleukin (IL)-2. The helper T cells also have antigen-presenting ability via CD40 ligand (CD40L)/CD40 pathway and enhance the production of antigen-specific immunoglobulin (Ig) G antibody in B cells.

The regenerated T cell population having the repertoire diversity of the present invention can act on any antigen or tissue. In particular, when the regenerated T cell population of the present invention is used for T cell replenishment therapy, it is preferable that the T cell population collected has specificity to cancer or tumor to be treated, tumor-related antigen, or its mutant antigen or virus. Cancer or tumor to be treated include, but are not limited thereto, breast cancer, lung cancer, liver cancer, oral cancer, upper pharyngeal cancer, head cervical cancer, gastric cancer, esophageal cancer, colon cancer, skin cancer, malignant melanoma, renal cancer, pancreatic cancer, brain tumor, prostate cancer, ovarian cancer, cervical cancer, colorectal cancer, bladder cancer, synovial sarcoma, leukemia, malignant lymphoma and multiple myeloma.

Examples of the tumor-related antigens or mutant antigens thereof include, but are not limited thereto, those which express specifically or non-specifically to each tumor, such as WT1, GPC3, XAGE1, MUC1, MUC5AC, MUC6, EGFRvIII, HER-2/neu, MAGE A3, MAGE A1, telomerase, PRAME, SSX2/4, PSCA, CTLA-4, gp100, GD2, GD3, fucosyl GM1, GM3, sLe (a), glycolipid F77, mesothelin, PD-L1, trp1, trp2, CD19, CD20, CD22, ROR1, CD33, c-Met, p53, p53 mutant, NY-ESO-1, PSMA, ETV6-AML, CEA, PSA, AFP, hTERT, EpCAM, ALK, androgen receptor, EphA2, CYP1B1, OY-TES-1, MAD-CT-2, MelanA/MART1, survivin, Ras, Ras mutant, ERG, bcr-ab1, XBP 1, and neoantigen caused by gene mutation and abnormal splice. Besides, examples of the virus antigen include, but are not limited thereto, inactivated viruses such as inactivated HBV and HPV, as well as proteins derived from various viruses such as EBVLMP1, EBV LMP2, EBNA (EBV nuclear antigen), HPV E1, HPV E2, HPV E6, HPV E7, HBV HBs, HTLV-1 Tax, and HBZ (HTLV-1 bZIP factor), etc. Further, in case of protein antigen, it may be a peptide fragment.

A biological tissue in the step 1 of the present invention refers to tissues of a mammal, particularly a human epithelial tissue, connective tissue, muscle tissue, neural tissue, body fluid, and the like. A biological tissue containing tumor-related antigens includes a cancer primary tissue and a cancer metastasis tissue. These tissues can be physically crushed into crushed materials by a homogenizer, an ultrasonic generator, or a freeze crusher, etc. Besides, these tissues can be dissolved into lysates by a solvent such as a phosphate-balanced buffer or by a surfactant such as CHAPS, which is an amphoteric surfactant, or Triton X and Tween 20, which are nonionic surfactants.

A very wide variety of T cells exist in vivo, so as to be able to react with any antigen. However, most of them are T cells irrelevant to the target antigens in T cell replenishment therapy. Accordingly, as a raw material for T cells produced through initialization to iPS cells, a T cell population which is reactive with cancer, tumor or virus to be treated by T cell replenishment therapy is preliminarily enriched, so that the therapeutic effect by the regenerated T cell population can be remarkably increased, and at the same time, immune response to unexpected antigens can be suppressed to improve safety.

In one embodiment of the present invention, the step of enriching a T cell population in step 1 comprises contacting one or more tumor-related antigens to activate and separating the proliferated T cells. When T cells are brought into contact with tumor-related antigens, T cell receptors recognize tumor-related antigens, and receive costimulatory signals, so that the T cells are activated. As a method for the contacting, the culture of peripheral blood mononuclear cells containing antigen presenting cells and T cells is carried out by using, for example, a culture solution added with a tumor-related antigen, a biological tissue containing a tumor-related antigen, or a crushed material or lysate thereof. Target T cells can be amplified efficiently by adding cytokines such as IL-2, IL-7, IL-15 and IL-21 into the culture solution. By adding the stimulus for enriching T cells having specificity to tumor-related antigens after the amplification culture, the activated T cells express functional molecules such as a CD137 molecule or IFN-γ on the surface of cell membrane. These functional molecules are stained with a specific antibody labeled with a fluorescent protein or magnetic bead, sorted by using a flow cytometer, or separated by using a magnet, so that the T cells having specificity to target tumor-related antigen can be enriched.

In the step 1 of the present invention, "having specificity to a tumor-related antigen" refers to that individual T cell specifically recognizes separate antigen epitope or peptide sequence, respectively while showing an immune response to a target tissue or antigen as a T cell population.

In one embodiment of the present invention, as a method for enriching a T cell population having specificity to target tissue or antigen, a method of contacting an antigen protein or peptide with T cells, activating a T cell population, and separating the proliferated T cells can be exemplified. In this method, a mononuclear cell fraction containing antigen presenting cells is cultured, and the antigen protein or peptide is added into the culture solution. By repeatedly bringing antigen protein or peptide into contact with T cells, the reactive T cells are proliferated, and their ratio to the whole T cell population is increased. On the other hand, the T cells which do not react with the added antigen protein or peptide are not activated, decrease gradually in ratio during the in-vitro culture, and finally die out. Thus, after culturing for a certain period, the T cell population having diversity in the antigen epitope or peptide sequence to be recognized while having reactivity specific to the antigen protein or peptide added in the culture solution is enriched and obtained.

In one embodiment of the present invention, as methods for enriching a T cell population, a method of selecting CD137 expression T cells and a method of selecting IFN-$\gamma$-producing T cells after activation of a T cell population can be exemplified. In these methods, antibodies specific to CD137 or IFN-$\gamma$ can be bound and subjected to magnetic separation using magnetic beads, or selective separation by a flow cytometer using fluorescent labeling. A system for capturing IFN-$\gamma$-producing cells with bispecific antibodies (bispecific antibodies against T cells and IFN-$\gamma$) may also be used. Moreover, in another embodiment of the present invention, a method of selecting T cells to which an oligomer of an antigen peptide-HLA (human leukocyte antigen) complex binds can be exemplified. In this method, an oligomer consisting of a complex of a specific HLA type and a target antigen peptide capable of presenting the HLA is labeled with a fluorescent dye. The cells bound with the oligomer are sorted using a flow cytometer, so that the T cells having specificity to target tissue or antigen can be obtained. When enriching the T cell population, these methods may be used alone or in combination.

[STEP 2]

In step 2 of the present invention, the enriched T cell population is initialized to iPS cells, and cultured while maintaining the repertoire diversity of the enriched T cell population.

Methods for producing iPS cells are well known in the art. In the present invention, the iPS cells are preferably produced by introducing an initialization factor into the T cell population enriched in the step 1. Here, as an initialization factor, a gene or gene product such as Oct3/4, Sox2, Sox1, Sox 3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, or the like can be exemplified. These initialization factors may be used alone or in combination.

The T cell population present in living body has a variety of TCR gene sequences as a population and has TCR repertoire diversity in this sense. iPS cells established from the T cell population are cultured while maintaining the repertoire diversity. When the iPS cells are cultured, a passage is preferably carried out. However, in a general passage method, since other cells except those to be used for passage are discarded, the diversity of the iPS cells established from the T cell population having repertoire diversity is gradually lost. In contrast, in the present invention, after establishing iPS cells, the culture vessel is preferably washed. Other cells except iPS cells are preferably removed. The iPS cells are recovered without cloning, and passaged in another culture vessel. The passage is performed repeatedly.

The culture solution used in the step 2 of the present invention is not particularly limited. However, a culture solution used for culturing animal cells can be used as a basic culture solution, to which cytokines for maintaining the undifferentiated ability of iPS cells can be added to prepare the culture solution. As the basic culture solution, for example, Iscove's Modified Dulbecue's Medium (IMDM) culture solution, Medium 199 culture solution, Eagle's Minimum Essential Medium (EMEM) culture solution, $\alpha$MEM culture solution, Dulbecco's modified Eagle's Medium (DMEM) culture solution, Ham's F12 culture solution, RPMI 1640 culture solution, Fischer's culture solution, Neurobasal Medium (Life Technologies Company), StemFit®, AK03N (Ajinomoto Healthy Supply), and a mixed culture solution thereof can be exemplified. Serum may be added to the culture solution. Otherwise, it may be serum-free. As cytokines, bFGF can be preferably exemplified. The concentration thereof in a culture solution is, for example, 1-100 $\mu$g/mL (preferably 50 $\mu$g/mL).

In one embodiment of the present invention, a method for culturing iPS cells may be an adhesion culture or a floating culture. However, adhesion culture is preferable. As a method for separating iPS cells, a method for separating dynamically by a cell scraper or the like, and a separation method using a separation solution having protease activity, a separation solution having collagenase activity, or a separation solution having protease activity and collagenase activity (for example, Accutase® and Accumax®, etc.) can be exemplified.

In one embodiment of the present invention, iPS cells are preferably passaged to another culture vessel when their cell density of $1 \times 10^3$ to $1 \times 10^4$ cells/cm$^2$, $1 \times 10^4$ to $1 \times 10^5$ cells/cm$^2$ or $1 \times 10^5$ to $1 \times 10^6$ cells/cm$^2$ is reached.

The number of passages may be any times as long as an amount of iPS cells required for T cell replenishment therapy are obtained, preferably 1 to 5 times or 5 to 10 times.

In one embodiment of the present invention, the iPS cells having the repertoire diversity obtained may be used as they are or cryopreserved until needed. Methods for cryopreservation of the cells are well known to those skilled in the art.

[STEP 3]

In step 3 of the present invention, a regenerated T cell population is produced from the cultured iPS cells. In one embodiment of the invention, the regenerated T cells maintain the repertoire diversity of the T cell population enriched in step 1 and have specificity to the tumor-related antigen used in step 1.

In one embodiment of the present invention, the regenerated T cell population is preferably obtained from the iPS cells cultured in step 2 of the present invention by differentiating into CD4/CD8 double-positive T cells via hematopoietic progenitor cells or from the iPS cells cultured in step 2 of the present invention by differentiating into CD8 single-positive T cells via hematopoietic progenitor cells and CD4/CD8 double-positive T cells.

Hematopoietic progenitor cells (HPC) are cells capable of differentiating into blood cells such as lymphocytes, eosinophils, neutrophils, basophils, erythrocytes, or megakaryocytes. In one embodiment of the present invention, hematopoietic progenitor cells and hematopoietic stem cells are not distinguished and refer to the same cells unless otherwise indicated. Hematopoietic stem cells/progenitor cells are recognized, for example, by that surface antigens CD34 and/or CD43 are positive.

In one embodiment of the present invention, the hematopoietic progenitor cells are preferably produced by culturing iPS cells in a culture solution added with vitamin C. The term "vitamin C" refers to L-ascorbic acid and its derivatives. An "L-ascorbic acid derivative" means a substance which converts into vitamin C by an enzymatic reaction in vivo. As examples of L-ascorbic acid derivatives, phosphate vitamin C, ascorbic acid glucoside, ascorbyl ethyl, vitamin C ester, ascorbyl tetraisopalmitate, ascorbyl stearate and ascorbic acid-2 phosphoric acid-6 palmitic acid can be exemplified. The L-ascorbic acid derivative is preferably vitamin C. For example, a phosphoric acid-L ascorbate such as phosphoric acid-sodium L ascorbate or phosphoric acid-magnesium L ascorbate can be exemplified. A vitamin C is contained in a culture solution at a concentration of, for example, 5-500 μg/mL.

In one embodiment of the present invention, a culture solution used for the production of hematopoietic progenitor cells is not particularly limited. It can be prepared by using a culture solution used for culturing animal cells as a basic culture solution, and adding vitamin C or the like thereto. As a basic culture solution, for example, an Iscove's Modified Dulbecco's Medium (IMDM) culture solution, a Medium 199 culture solution, an Eagle's Minimum Essential Medium (EMEM) culture solution, an αMEM culture solution, Dulbecco's modified Eagle's Medium (DMEM) culture solution, Ham's F12 culture solution, RPMI 1640 culture solution, Fischer's culture solution, Neurobasal Medium (Life Technologies), StemPro34 (Life Technologies), and their mixed culture solutions can be exemplified. The culture solution may contain serum or may be serum-free. If necessary, the basic culture solution may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, cytokines, and the like.

In one embodiment of the present invention, a cytokine selected from the group consisting of BMP4 (bone morphogenetic protein 4), VEGF (vascular endothelial growth factor), bFGF (basic fibroblast growth factor), SCF (stem cell factor), TPO (thrombopoietin), and FLT-3L (Flt3 ligand) may be furtherly added to the culture solution used for producing hematopoietic progenitor cells.

In one embodiment of the present invention, their concentrations are, for example, 1-100 ng/mL for BMP4, 1-100 ng/mL for VEGF, 1-100 ng/mL for bFGF, 10-100 ng/mL for SCF, 1-100 ng/mL for TPO, and 1-100 ng/mL for FLT-3L.

In one embodiment of the present invention, a TGFβ inhibitor may be added to the culture solution. The term "TGFβ inhibitor" is a small-molecular inhibitor interfering with the signaling of TGFβ family. For example, SB431542 and SB202190 (R. K. Lindemann et al., Mol. Cancer 2: 20(2003), SB505124 (GlaxoSmithKline), NPC30345, SD093, SD908, and SD208 (Scios), and LY2109761, LY364947 and LY580276 (Lilly Research Laboratories) can be exemplified. They can be added to the culture solution at a concentration of preferably 0.5-100 μM.

In one embodiment of the present invention, iPS cells can be co-cultured with feeder cells such as C3H10T1/2 (Takayama N. et al. J Exp Med. 2817-2830, 2010), or stroma cells derived from different types (Niwa A et al. J Cell Physiol. 2009 Nov; 221 (2): 3677-77). However, iPS cells may be cultured preferably without feeder cells.

In one embodiment of the present invention, the method for culturing iPS cells in the production of hematopoietic progenitor cells can be an adhesion culture or a floating culture, preferably the floating culture. For example, iPS cells can be subjected to the floating culture after separating the cultured colonies until 80% confluent to the used dish and dissociating the colonies into single cells. As methods for separating iPS cells, a method for separating dynamically by a cell scraper or the like, and a separation method using a separation solution having protease activity and collagenase activity (for example, Accutase® and Accumax®, etc.) or a separation solution having collagenase activity can be exemplified.

Floating culture is a culture performed under a state where cells are not adhesive to culture vessel. In one embodiment of the present invention, a floating culture can be performed, but is not particularly limited thereto, by using a culture vessel which is not artificially treated for the purpose of improving adhesion to cells (for example, coating treatment by an extracellular matrix or the like), or a culture vessel which is artificially treated to suppress the adhesion (for example, coating treatment by a polyhydroxyethyl methacrylate (poly-HEMA) or a nonionic surfactant (Pluronic F-127, etc.). During the floating culture, it is preferable to form an embryoid body (EB) and culture.

In another embodiment of the present invention, hematopoietic progenitor cells can also be prepared from net-like structures (also referred to as iPS-sac) obtained by culturing iPS cells. The "net-like structure" is a three-dimensional bursal structure derived from iPS cells, formed by an endothelial cell population or the like, and contains hematopoietic progenitor cells in the interior thereof.

In one embodiment of the present invention, the temperature condition during the culture for producing hematopoietic progenitor cells from iPS cells is not particularly limited. However, it is, for example, about 37°C to about 42°C, preferably about 37°C to about 39°C. In addition, a person skilled in the art can appropriately determine the culture period while monitoring the number of cells of hematopoietic progenitor cells or the like. The days of culturing is not particularly limited as long as the hematopoietic progenitor cells are obtained. However, it is, for example, at least 6 days or more, 7 days or more, 8 days or more, 9 days or more, 11 days or more, 12 days or more, 12 days or more, 13 days or more, or 14 days or more, and preferably 14 days. Long culture period is not considered as a problem in the production of hematopoietic progenitor cells. Besides, the culture may be performed under a condition of low oxygen. In one embodiment of the present invention, as conditions of low oxygen, for example, oxygen concentrations of 15%, 10%, 9%, 8%, 7%, 6%, 5% or less can be exemplified.

In the present invention, "CD4/CD8 double-positive T cells" refer to $CD8^+$ $CD4^+$ cells wherein CD4 and CD8, the surface antigen, among T cells are both positive. T cells can be recognized by being positive of CD3 and CD45 which are surface antigens. Accordingly, the CD4/CD8 double-positive T cells can be identified as a cell being positive of CD4, CD8, CD3 and CD45. CD4/CD8 double-positive T cells can be differentiated by induction into CD4 single-positive cells or CD8 single-positive cells.

In one embodiment of the invention, the CD8 double-positive T cell can be produced by a method comprising a step of culturing hematopoietic progenitor cells in a culture solution added with p38 inhibitor and/or SDF-1.

In the present invention, a "p38 inhibitor" is defined as a substance that inhibits the function of p38 protein (p38MAP kinase). In one embodiment of the present invention, as p38 inhibitors, for example, but are not limited thereto, chemical inhibitors of p38, dominant negative variants of p38, or nucleic acids encoding the same can be exemplified.

In one embodiment of the present invention, examples of chemical inhibitors of p38 include, but are not limited thereto, SB203580 (4-(4-fluorophenyl)-2-(4-methylsulfonylphenyl)-5-(4-pyridyl)-1H-imidazole) and its derivatives, SB202190 (4-(4-fluorophenyl)-2-(4-hydroxyphenyl)-5-(4-pyridyl)-1H-imidazole) and its derivatives, SB239063 (trans-4-[4-(4-fluorophenyl)-5-(2-methoxy-4-pyrimidinyl)-1H-imidazol-1-yl]cyclohexanol) and its derivatives, SB220025 and its derivatives, PD169316, RPR200765A, AMG-548, BIRB-796, SC10-469, SCIO-323, VX-702, or FR167653. These compounds are commercially available. For example, SB203580, SB202190, SC239063, SB220025 and PD169316 are available from Calbiochem, and SL10-469 and SCIO-323 are available from Scios.

In one embodiment of the present invention, as dominant negative variants of p38, p38T180A in which the threonine at position 180 located in the DNA binding region of p38 is point-mutated to alanine, and p38Y182F in which the tyrosine at position 182 of p38 in human and mouse is point-mutated to phenylalanine, and the like can be exemplified.

A p38 inhibitor is added to the culture solution in a concentration range of, for example, from about 1 μM to about 50 μM.

In one embodiment of the present invention, SDF-1 (stromal cell-derived factor 1) is not only SDF-1α or its mature form, but may also be an isoform such as SDF-1β, SDF-1γ, SDF-1δ, SDF-1ε or SDF-1φ, or a mature form thereof, or a mixture of any ratio thereof. Preferably, SDF-1α is used. The SDF-1 may also be referred to as CXCL-12 or PBSF.

In one embodiment of the present invention, the SDF-1 may be substituted with one or several amino acids in its amino acid sequence, deleted and/or added as long as it has the activity as chemokine. Similarly, its sugar chain may be substituted, deleted, and/or added. In SDF-1, if at least four cysteine residues (in case of human SDF-1α, Cys30, Cys32, Cys55 and Cys71) are maintained, and it has identity of 90% or more to the amino acid sequence of natural form, amino acid mutation is tolerated. The SDF-1 may be derived from a mammal such as a human, or a non-human mammal such as a monkey, sheep, cow, horse, pig, dog, cat, rabbit, rat, or mouse. For example, the protein registered with GenBank accession number of NP_954637 can be used as human SDF-1α, and the protein registered with GenBank accession number of NP_000600 can be used as SDF-1β.

In one embodiment of the present invention, as SDF-1, those which are commercially available, those which are purified from nature ones, or those which are produced by peptide synthesis or genetic engineering techniques may be used.

In one embodiment of the present invention, SDF-1 is added to a culture solution in a concentration range of, for example, about 10 ng/mL to about 100 ng/mL.

In one embodiment of the present invention, the culture solution used for the production of CD4/CD8 double-positive T cells is not particularly limited. It can be prepared by using a culture solution used for culturing animal cells as a basic culture solution, adding a p38 inhibitor and/or SDF-1, and preferably furtherly vitamin C thereto. The vitamin C used in the production process of the CD4/CD8 double-positive T cells is, for example, as described above. The concentration of vitamin C is, for example, 5-200 μg/mL. As the basic culture solution, for example, Iscove's Modified Dulbecco's Medium (IMDM) culture solution, Medium 199 culture solution, Eagle's Minimum Essential Medium (EMEM) culture solution, αMEM culture solution, Dulbecco's modified Eagle's Medium (DMEM) culture solution, Ham's F12 culture solution, RPMI 1640 culture solution, Fischer's culture solution, OP9 culture solution, Neurobasal Medium (Life Technologies), and their mixed culture solutions can be exemplified. The culture solution may be added with serum, or it may be serum-free. If necessary, the basic culture solution may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, and cytokines.

In one embodiment of the present invention, a cytokine selected from the group consisting of SCF, TPO (thrombopoietin), FLT-3L and IL-7 can be furtherly added to a culture solution used for producing CD4/CD8 double-positive T cells. Their concentrations are, for example, 10-100 ng/mL for SCF, 1-200 ng/mL for TPO, 1-100 ng/mL for FLT-3L, and 1-100 ng/mL for IL-7.

In one embodiment of the present invention, when producing the CD4/CD8 double-positive T cells, hematopoietic progenitor cells may be cultured using feeder cells. However, they may be preferably cultured without feeder cells.

In one embodiment of the present invention, the culture of hematopoietic progenitor cells may be adhesion culture or floating culture. However, in the present process, adhesion culture is preferable. In case of adhesion culture, a culture vessel may be coated for use. As a coating agent, for example, matrix gel (Niwa A, et al. PLoS One. 6(7): e22261, 2011), collagen, gelatin, laminin, heparan sulfate proteoglycan, retronectin, Fc-DLL4 or entactin, and combinations thereof can be exemplified.

In another embodiment of the present invention, when hematopoietic progenitor cells are obtained by floating culture of embryoid body, it is preferable to perform adhesion culture after dissociating them into single cells.

In one embodiment of the present invention, the culture temperature condition in the culture of the hematopoietic progenitor cells for producing CD4/CD8 double-positive T cells is not particularly limited. However, it is, for example, preferably about 37°C to about 42°C, and more preferably about 37°C to about 39°C. In addition, a person skilled in the art can appropriately determine the culture period while monitoring the number of CD4/CD8 double-positive T cells or the like. Days of culturing are not particularly limited as long as CD4/CD8 double-positive T cells are obtained. However, they are, for example, at least 10 days or more, 12 days or more, 14 days or more, 16 days or more, 18 days or more, 20 days or more, 22 days or more, or 23 days or more, and preferably 23 days.

In one embodiment of the present invention, the obtained CD4/CD8 double-positive T cells may be isolated, or used as a cell population containing other cell types. In the case of isolating, an index selected from the group consisting of CD4, CD8, CD3 and CD45 can be used in the isolation. Regarding the isolation methods, those well known to a person skilled in the art can be used. For example, an isolation method using a flow cytometer by labeling with an antibody against CD4, CD8, CD3 or CD45, or a method for purifying the desired antigen by using an immobilized affinity column can be exemplified.

"CD8 single-positive T cells" refer to CD8$^+$ CD4$^-$ cells wherein the surface antigen CD8 is positive. They are also referred to as cytotoxic T cells. Since T cells are recognized by that surface antigens CD4 and CD45 are positive, CD8 single-positive T cells can be identified by being CD8, CD3 and CD45 positive, and CD4 negative.

In one embodiment of the present invention, the CD8 single-positive T cells can be produced by a method comprising a step for culturing CD4/CD8 double-positive T cells in a culture solution added with adrenocortical hormone agent.

In one embodiment of the present invention, the adrenocortical hormone agent is preferably a carbohydrate corticoid or a derivative thereof. Their examples include cortisone acetate, hydrocortisone, fludrocortisone acetate, prednisolone, triamcinolone, methylprednisolone, dexamethasone, betamethasone, or beclomethasone propionate. Preferably, the adrenocortical hormone agent is dexamethasone. Its concentration in a culture solution is, for example, 1-100 nM

In one embodiment of the present invention, the culture solution used for producing CD8 single-positive T cells is not particularly limited. However, it can be prepared by using a culture solution used for culturing animal cells as a basic culture solution, and adding an adrenocortical hormone agent thereto. As a basic culture solution, for example, Iscove's Modified Dulbecco's Medium (IMDM) culture solution, Medium 199 culture solution, Eagle's Minimum Essential Medium (EMEM) culture solution, αMEM culture solution, Dulbecco's modified Eagle's Medium (DMEM) culture solution, Ham's F12 culture solution, RPMI 1640 culture solution, Fischer's culture solution, Neurobasal Medium (Life Technologies), and their mixed culture solutions can be exemplified. The culture solution may be added with serum, or may be serum-free. If necessary, the basic culture solution may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, and cytokines, etc.

In one embodiment of the present invention, it is preferable that the culture solution used in the production of CD8 single-positive T cells further contains an anti-CD3 antibody, vitamin C, or cytokines. Examples of the cytokine include IL-2 and IL-7, etc.

In one embodiment of the present invention, the anti-CD3 antibody is not particularly limited as long as it is an antibody that specifically recognizes CD3. For example, an antibody produced from an OKT3 clone can be exemplified. The concentration of the anti-CD3 antibody in a culture solution is, for example, 10-1000 ng/mL.

In one embodiment of the present invention, the vitamin C used in the production of CD8 single-positive T cells is, for example, the above-described ones, and can be used under the same conditions as described above.

In one embodiment of the present invention, the concentration of cytokine used in the production of CD8 single-positive T cells is, for example, 10-1000 U/mL for IL-2, and 1-100 ng/mL for IL-7.

In one embodiment of the present invention, the temperature condition in culturing the CD4/CD8 double-positive T cells for the production of CD8 single-positive T cells is not particularly limited. It is, for example, preferably about 37°C to about 42°C, and more preferably about 37°C to about 39°C. In addition, a person skilled in the art can appropriately determine the culture period while monitoring the number of CD8 single-positive T cells or the like. Days of culturing are not particularly limited as long as the CD8 single-positive T cells are obtained. However, they are, for example, at least 1 day or more, 2 days or more, 3 days or more, 4 days or more, or 5 days or more, and preferably 3 days.

In one embodiment of the present invention, regarding the degree of repertoire diversity to be maintained, preferably two or more, more preferably three or more, and even more preferably four or more types of Vβ are maintained.

In one embodiment of the present invention, the regenerated T cell population produced is preferably used in T cell replenishment therapy. In the present invention, T cell replenishment therapy means a therapy wherein T cells are replenished to a living body. In one embodiment of the present invention, T cell replenishment therapy is preferably a therapy by cell transplantation including infusion, intratumoral injection, intraarterial injection, portal vein injection, etc. of regenerated T cells.

In the case where a regenerated T cell population obtained by the present invention is used for T cell replenishment therapy, for lowing the possibility of rejection, it is preferable that the cancer patient or non-cancer patient from whom T cells are collected has the same type of HLA as the cancer patient or a cancer patient to whom the T cells obtained by the present invention are administered for the treatment of cancer has. Moreover, it is more preferable that a cancer patient to whom T cells obtained by the present invention are administered is the same cancer patient from whom the T cells are collected. That is, cancer treatment by autologous transplantation is more preferable.

The present invention includes a regenerated T cell population which is obtained via iPS cells and maintains repertoire diversity of a T cell population present in a living body. The pharmaceutical composition containing the regenerated T cell population of the present invention can be used to treat a cancer treatment subject. One embodiment of the present invention includes a method for treating cancer using the pharmaceutical composition of the present invention. The pharmaceutical composition of the present invention may contain a pharmaceutically acceptable additive. As the additive, cell culture solution, phosphate buffered saline and the like can be exemplified.

The present invention is described more specifically using the following examples. However, the scope of the present invention is not limited by these examples.

## EXAMPLES

[Specific procedure of step 1]

A tumor-related antigen was added to a culture solution, brought into contact with a T-cell population, and cultured for 12 days in RPMI-1640 culture solution (containing 10% human AB serum and 1% penicillin-streptomycin-glutamine) added with 200 U/mL IL-2, 10 ng/mL IL-15 and 10 ng/mL IL-21. In case that the tumor-related antigen to be added to the culture solution is a peptide, it is preferably added at a concentration of 1-100 mg/mL. In case that a biological tissue or a crushed material thereof is used as the tumor-related antigen, it is preferable that it is be shredded into squares of 1-2 mm, and that 1 to 5 pieces of them are added to a tube containing the culture solution to be brought into contact with a T-cell population. In case of lysate a biological tissue, the lysate is preferably added to the culture solution and brought into contact with the T cell population. On 12 days after the culture, the same tumor-related antigen peptide was brought into contact again and cultured further for 24 hours to express an activation marker for T-cell cells having specificity to the tumor-related antigen. For the cultured cell population, negative selection using a blood cell fraction marker except a CD8 molecule (CD8+ T cell isolation kit, Miltenyi Biotech) and positive selection to CD137 or IFN-γ as an activation marker (CD137 MicorBead Kit or Cytokine capture system-IFN-y, Miltenyi Biotech) were performed, so that only CD8 single-positive T cells having specificity to the tumor-related antigen were enriched.

In the case of using the antigen peptide-HLA complex in step 1, the T cell population to be used may be either a cell population which are brought into contact with the tumor-related antigen and cultured for 12 days, or a cell population which are not cultured. A fluorescently labeled target antigen peptide-HLA complex was added to the obtained cell population and stained for 30 minutes. Then, a fluorescently labeled antibody against other blood cell markers (CD8, CD4 and CD14) was added, and stained further for 30 minutes. After the staining, the collected cells were washed with phosphate buffer. Only the cells which express the antigen peptide-HLA complex and CD8 molecules were sorted by using a flow cytometer, so that T cells having specificity to the target tumor-related antigen were enriched.

[Specific procedure of step 2: Initialization to iPS cells]

Subsequently, T cells were recovered to a 15 mL tube, suspended in about 250 μL of RPMI-1640 culture solution. Then, Sendai virus containing four essential factors (Oct3/4, Sox2, Klf4 and c-Myc) for the initialization at titer of MOI=5 was added to the culture solution using a CytoTune-iPS 2.0 kit (ID Pharma, catalog number DV-0304)) and incubated at 37°C for 2 hours. After the incubation, the T cells were washed with RPMI-1640 culture solution. The virus was removed from the culture solution.

Subsequently, in order to remove magnetic beads, the beads and the cells were separated by pipetting. The tube was let stand still on a stand equipped with magnet, so that only the T cells floating in the culture solution were recovered.

The obtained T-cells were seeded on a 6-cm dish coated with iMatrix-511 (Nippi, catalog number 892011) at 0.5 μg/cm$^2$. Culture in a RPMI-1640 culture solution was started by using an incubator set at 37°C and 5% $CO_2$.

From the next day of starting of the culture on the 6-cm dish, half of the cell-free culture supernatant was removed. A culture solution for establishment wherein 1 mM valproic acid was added to the StemFit® AK03N (Ajinomoto Healthy Supply Co., Inc.) was added in the same amount as that of the removed culture solution.

Thereafter, while the same exchange of half-amount of culture solution was conducted daily, the culture was continued for 20-40 days. Accordingly, a large number of iPS cell colonies were obtained in the 6-cm dish. In the stage where a certain amount of colonies had been obtained, the culture was continued by changing from the culture solution for establishment to the culture solution for iPS cells (only the StemFit AK03N).

[Specific procedure of step 2: Culturing while maintaining the repertoire diversity of established iPS cells]

In a stage where colonies of iPS cells had grown to such a degree that the colonies of iPS cells could be confirmed by naked eyes, iPS cells were passaged to a 6-cm dish newly coated with iMatrix-511.

In the passage, the iPS cells adhered to the dish were washed with PBS (-), then added with TrypLeSelect (Life Technologies, A12859-01), and incubated for about 7 minutes to peel the iPS cells and dispersed into single cells. The cells were washed with the culture solution for iPS cells. The number of cells was counted. The dispersed cells were then seeded at a density of 1500 cells/cm$^2$ in a 6-cm dish newly coated with iMatrix-511. All of the recovered iPS cells were seeded into a new dish without being discarded.

By planting all the recovered cells, the culture scale gradually expanded. When 3 to 5 passages had been performed after establishment, and iPS cells were obtained in an amount necessary for T cell replenishment therapy using iPS cells, the cells were cryopreserved according to following procedures.

After the iPS cells were recovered according to the same procedure as that for the passage, the cells were floated in a cell frozen liquid such as a TC protector (DS Pharma, catalog number KBTCP001). The temperature was decreased to -80°C at a speed of - 1°C/minute, so that the iPS cells were frozen. For long term and stable preservation of the iPS cells, the preservation in liquid nitrogen (-196°C) is desirable.

[Specific procedure of step 3]

The iPS cells obtained in the step 2 were seeded (Day 0) at $3 \times 10^5$-$6 \times 10^5$ cells/well in a 6-well plate (Corning, catalog number 3471) treated for ultra-low adhesion. 10 ng/mL BMP4, 5 ng/mL bFGF, 15 ng/mL VEGF and 2 μM SB431542 were added to an EB culture solution (StemPro34 containing 10 μg/mL human insulin, 5.5 μg/mL human transferrin, 5 ng/mL sodium selenate, 2 mM L-glutamine, 45 mM α-monothioglycerol, and 50 μg/mL ascorbic acid), and cultured under low oxygen condition (5% $O_2$) for 5 days (Day 5).

Then, 50 ng/mL SCF, 30 ng/mL TPO, and 10 ng/mL Flt3L were added and cultured further for 5-9 days (up to Day 14).

The obtained hematopoietic progenitor cells were cultured for 21 days (Day 35) on a 4-well plate coated with Fc-DLL4 (5 μg/mL) (Sino Biological Inc.) and Retronectin (5 μg/mL) (Takara Bio Inc.) in an OP9 culture solution (containing 15% FBS, 2 mM L-glutamine, 100 U/mL penicillin, 100 ng/mL streptomycin, 55 $\mu$M 2-mercaptoethanol, 50 $\mu$g/mL ascorbic acid, 10 $\mu$g/mL human insulin, 5.5 $\mu$g/mL human transferrin, and 5 ng/mL sodium selenite) added with 50 ng/mL SCF, 50 ng/mL IL-7, 50 ng/mL Flt3L, 100 ng/mL TPO, 15 $\mu$M SB203580 (Tocris Bioscience) and 30 ng/mL SDF-1$\alpha$.

On day 35, the fraction wherein all of CD45, CD3, CD4 and CD8 were positive were isolated by using FACS. CD4/CD8 double-positive cells (referred to as DP cells) were obtained.

The obtained CD4/CD8 double-positive cells were cultured for 2 days (Day 37) on a 96-well plate in RPMI 1640 culture solution added with 15% fetal bovine serum, 500 ng/mL anti-CD3 antibody (eBioscience), 200 U/mL IL-2, 10 ng/mL IL-7 and 10 nM dexamethasone. The cells were washed with RPMI 1640 culture solution added with 15% fetal bovine serum to remove the antibody, and cultured further for 5 days with RPMI 1640 culture solution added with 15% fetal bovine serum and 10 ng/mL IL-7. CD8 single-positive T cells were obtained (Day 42).

## Example 1

According to the above method, iPS cells were established from a T cell population having repertoire diversity separated from extirpated tumor of a patient with lung cancer. Re-differentiation induction to T cells having the repertoire diversity was performed.

The repertoire diversity of a T cell receptor (TCR) expressed by T-cell is generally determined by sequence analysis of DNA and RNA. However, it can also be easily detected by using a panel of antibody which specifically binds to each segment of V-$\beta$ chain of the TCR.

TCR repertoire of a T cell population separated from extirpated tumor of a patient with lung cancer was analyzed (FIG. 1) by using a TCRV$\beta$ analysis kit (Beckman Coulter, catalog number IM-3497). It was confirmed that cells expressing the respective V$\beta$-chains were contained in the specimen of the patient at a rate of about 1-13% and they were T cell populations having repertoire diversity. These T cell populations were initialized to iPS cells according to the above method.

iPS cells are pluripotent stem cells, wherein a T cell-related gene containing a TCR gene is not expressed. That is, it is difficult to analyze the diversity of TCR genes at the stage of undifferentiated iPS cells. The established iPS cells were passaged so as not to lose repertoire diversity, and then subjected to differentiation induction to T cells via a stage of hematopoietic progenitor cell.

The repertoire of TCR expressing the regenerated T cells was analyzed (FIG. 2) by using the kit described above. It was confirmed that the diversity of T cells expressing the segments of 2, 3, 4, 7.1, 7.2, 8, 12, 14, 16, 20 and 21.3 of V$\beta$ was lost with the establishment of iPS cells and the differentiation induction of T cells, while about half of the V$\beta$ repertoire was remained, and the regenerated T cells were the T cell populations having repertoire diversity.

## Example 2

Peripheral blood was collected from a patient in to whom a peptide vaccine was administered against a GPC3, a tumor antigen, and the mononuclear cell population was separated by using a density gradient centrifugation method. $2\times10^6$ cells of the cell population was added to a 24-well plate. T cells having specificity to GPC3 were amplified and cultured according to the "Specific procedure of step 1" described above using 10 $\mu$g/mL GPC3 peptide. The cultured cells were again brought into contact with the GPC3 peptide again and cultured for 24 hours to express activation marker. The cell population was stained with an antibody (CD4-BV421, CD8-PerCP/Cy5.5, CD14-FITC and CD137-PE). The ratio of a T cell population having GPC3 specificity was analyzed (FIG. 3) by using a flow cytometer (BD FACSAria® II). As a result, it was shown that the CD8/CD137 double-positive cells having specificity to GPC3 were amplified at a high rate. For this cell population, T cell populations which express both CD8 and CD137 and have specificity to GPC3 were enriched by using magnetic beads (FIG. 4). The cell populations were initialized to iPS cells according to the "Specific procedure of step 2 (Initialization to iPS cells and Culture while maintaining the repertoire diversity of established iPS cells)" described above, and re-differentiation induction to CD8 single-positive cells was carried out according to the "Specific procedure of step 3" described above. Since the obtained regenerated CD8 single-positive cells were stained at a high rate to GPC3 peptide-HLA complex (dextramer), they were shown to be T cell population having specificity to GPC3 (FIG. 5).

## Example 3

Mononuclear cells were separated from peripheral blood of a non-cancer patient (healthy subject) and brought into contact with an overwrap peptide of WT1 (PepTivator WT1, Miltenyi Biotech), a tumor antigen, so that a T cell population having specificity to WT1 was amplified and cultured in the same manner as in Example 2. The WT1 overwrap peptide was added to the culture solution at 1 $\mu$g/mL. The activation marker was expressed by bringing the cultured cells again into contact with the WT1 overwrap peptide. As a result of the analysis, the CD8/CD137 double-positive cells which have specificity to WT1 were shown to be amplified at a high rate (FIG. 6). For this cell population, T cell populations expressing both CD8 and CD137, and having specificity to WT1 were enriched (FIG. 7) by using magnetic beads.

**Example 4**

Mononuclear cells were separated from peripheral blood of a non-cancer patient (healthy subject) and brought into contact with an overwrap peptide of EBV LMP2A (PepTivator EBV LMP2A, Miltenyi Biotech), a virus antigen, so that a T cell population having specificity to EBV LMP2A was amplified and cultured in the same manner as in Example 2. The EBV LMP2A overwrap peptide was added to the culture solution at 1 μg/mL. The activation marker was expressed by bringing the cultured cells again into contact with the EBV LMP2A overwrap peptide. As a result of the analysis, the CD8/CD137 double-positive cells which have specificity to EBV LMP2A were shown to be amplified at a high rate (FIG. 8). For this cell population, T cell populations expressing both CD8 and CD137, and having specificity to EBV LMP2A were enriched (FIG. 9) by using magnetic beads.

**Example 5**

Aregenerated T cell population produced via iPS cells from a T cell population having specificity to GPC3 was confirmed to have specific cytotoxic activity to target cells expressing the GPC3 peptide.

Mononuclear cells were separated from peripheral blood of a patient with liver cancer, and brought into contact with GPC3 peptide in the same manner as in Example 2, so that a T cell population having specificity to GPC3 was amplified and cultured, and further enriched. The enriched cell population was initialized to iPS cells according to the above steps 2 and 3. Then, re-differentiation induction to CD8 single-positive cells was carried out according to the above step 4. The obtained regenerated T cell population was analyzed by flow cytometry. It was confirmed that CD8 was present as CD8αβ heterodimer (FIG. 10A).

B cells infected and immortalized with EB virus were used as target cells. The target cells were cultured in the presence or absence of GPC3 peptide (1 μM). Cells which express GPC3 peptide and cells which do not express GPC3 peptide were produced. Cytotoxic activity of a regenerated T cell population to the target cells was determined by using an N-SPC non-RI cytotoxic assay kit (Techno Suzuta). The cells which express GPC3 peptide and cells which do not express GPC3 peptide were treated with BM-HT reagent. The BM-HT reagent was incorporated into the cells. The target cells were then washed and excess BM-HT reagent was removed. The target cells and the regenerated T cell population were co-cultured for 2 hours. The culture supernatant was added to a europium (Eu) solution. The HT chelate leaking into the culture solution from the damaged target cells turned into HT/EU complex, which was excited by a laser beam, and the cytotoxic activity was assessed by measuring time-resolved fluorescence. The cytotoxic activity was calculated by the following formula:

$$\text{Cytotoxic activity (\%)} = [\text{fluorescence amount in the culture supernatant in the presence of regenerated T cells - fluorescence amount in the culture supernatant in the absence of regenerated T cells}] \, / \, [\text{fluorescence amount in the solution of total target cells - fluorescence amount in the culture supernatant in the absence of regenerated T cells}] \times 100$$

The ratio of the cell numbers of the regenerated T cells to the target cells in co-culture was taken as 20:1, 10:1 and 5:1. As a result, the cytotoxic activity of the regenerated T cell population to target cells expressing the GPC3 peptide was shown. The activity was enhanced in response to an increase in the cell number of the regenerated T cells with respect to the target cells (FIG. 10B). On the other hand, the regenerated T cell population showed no cytotoxic activity to the target cells which did not express GPC3 peptide (FIG. 10B). From these results, it is shown that the obtained regenerated T cell population maintains antigen specificity for GPC3 and has antigen-specific cytotoxic activity to target cells expressing GPC3.

**Claims**

1. A method for producing a regenerated T cell population via iPS cells, comprising the steps of:

   step 1: bringing a T cell population into contact with one or more tumor-related antigens, or a biological tissue containing one or more tumor-related antigens, or a crushed material or a lysate thereof, and enriching the T cell population having specificity to the tumor-related antigens,

step 2: initializing the enriched T cell population to iPS cells, culturing while maintaining the repertoire diversity of the enriched T cell population, and

step 3: producing a regenerated T cell population from the cultured iPS cells.

2. The method according to claim 1, wherein the regenerated T cell population maintains the repertoire diversity of the enriched T cell population and has specificity to the tumor-related antigens.

3. The method according to claim 1 or 2, wherein the T cell population in step 1 is derived from a mammal.

4. The method according to claim 3, wherein the mammal is a human.

5. The method according to claim 4, wherein the human is a cancer patient or a non-cancer patient.

6. The method according to claim 5, wherein the cancer patient or the non-cancer patient has been administered, or currently being administered, or scheduled to be administered cancer vaccine.

7. The method according to any of claims 1 to 6, wherein the T cell population in step 1 is derived from body fluid.

8. The method according to claim 7, wherein the body fluid is selected from the group consisting of blood, lymph fluid, tissue fluid, body cavity fluid (including ascites, pleural fluid, pericardial fluid, cerebrospinal fluid, joint fluid and aqueous humor), nasal juice and urine.

9. The method according to any of claims 1 to 6, wherein the T cell population in step 1 is T cells derived from a tumor tissue.

10. The method according to any of claims 1 to 9, wherein the T cell population in step 1 is $\alpha\beta$T cells, $\gamma\delta$ T cells, helper T cells, regular T cells, cytotoxic T cells, natural killer T cells, or mixtures thereof.

11. The method according to any of claims 1 to 10, wherein the biological tissue in step 1 is a cancer primary tissue and a cancer metastasis tissue.

12. The method according to any of claims 1 to 11, wherein the step of enriching the T cell population in step 1 comprises steps of activating by contacting with one or more tumor-related antigens, and separating the proliferated T cells.

13. The method according to any of claims 1 to 12, wherein the step of enriching the T cell population in step 1 comprises one or more steps selected from the group consisting of a step of selecting CD137 expression T cells and a step of selecting IFN-$\gamma$ producing T cells from the T cell population.

14. The method according to any of claims 1 to 12, wherein the step of enriching the T cell population in step 1 comprises one or more steps selected from the group consisting of a step of selecting CDS137 expression T cells, a step of selecting IFN-$\gamma$ producing T cells, and a step of selecting T cells to which the antigen peptide-HLA complex binds from the T cell population.

15. The method according to any of claims 1 to 14, wherein the step 2 comprises recovering iPS cells without cloning and passaging.

16. The method according to any of claims 1 to 15, wherein the regenerated T cell population obtained in step 3 is used for T cell replenishment therapy.

17. The method according to any of claims 1 to 16, wherein the type of V$\beta$ to be maintained by the regenerated T cell population is two or more.

18. A regenerated T cell population produced by the method according to any of claims 1 to 17.

19. The regenerated T cell population according to claim 18, which maintains repertoire diversity of T cell population present in living body.

20. A pharmaceutical composition which contains the regenerated T cell population according to claim 18 or 19.

21. The pharmaceutical composition according to claim 20, for treating a cancer treatment subject by autologous or allogeneic transplantation.

22. A method for treating cancer which uses the pharmaceutical composition according to claim 20 or 21.

Figure 1

Figure 2

Figure 3

CD4(-)CD14(-) fraction

Figure 4

CD4(-)CD14(-) fraction

Figure 5

CD45(+) fraction    CD45(+) fraction    CD45(+)CD8(+) fraction

Figure 6

CD4(-)CD14(-) fraction

Figure 7

CD4(-)CD14(-) fraction

Figure 8

CD4(-)CD14(-) fraction

Figure 9

CD4(-)CD14(-) fraction

Figure 10

（A）

# CD8αβ positive regenerated T cells

（B）

Each point indicates average value± standard deviation

## INTERNATIONAL SEARCH REPORT

| International application No. |
| --- |
| PCT/JP2019/029750 |

**A. CLASSIFICATION OF SUBJECT MATTER**
Int.Cl.  C12N5/0783(2010.01)i, A61K35/17(2015.01)i, A61P35/00(2006.01)i,
C12N5/10(2006.01)i, A61K35/545(2015.01)n

According to International Patent Classification (IPC) or to both national classification and IPC

**B. FIELDS SEARCHED**

Minimum documentation searched (classification system followed by classification symbols)
Int.Cl. C12N5/0783, A61K35/17, A61P35/00, C12N5/10, A61K35/545

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2017/078807 A1 (FATE THERAPEUTICS, INC.) 11 May 2017, paragraphs [0383]–[0390] & JP 2018·504122 A paragraphs [0383]–[0390] & US 2018/0320137 A1 & EP 3371301 A1 & KR 10-2018-0066263 A & CN 108473961 A | 1–12, 15–22 |
| Y | | 13–14 |
| X | SAITO, Hidehito et al., "Reprogramming of Melanoma Tumor-Infiltrating Lymphocytes to Induced Pluripotent Stem Cells", Stem Cells International, 2016, vol. 2016, Article ID 8394960, Supplementary Material, abstract, page 1, right column, paragraph [0002], page 2, left column, paragraph [0002], page 2, right column, paragraphs [0003]–[0004], page 9, right column, paragraphs [0003]–[0005] | 18–22 |
| Y | | 1–22 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 13 September 2019 (13.09.2019) | 01 October 2019 (01.10.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/029750

C (Continuation).   DOCUMENTS CONSIDERED TO BE RELEVANT

| Category* | Citation of document, with indication, where appropriate, of the relevant passages | Relevant to claim No. |
|---|---|---|
| X | WO 2018/005712 A1 (GENEIUS BIOTECHNOLOGY, INC.) 04 | 18-22 |
| Y | January 2018, claims, paragraphs [0002]-[0005], [0007], [0074]-[0095], [0114]-[0128], [0144], [0149], [0160]-[0162] & EP 3487990 A1 & KR 10-2019-0037243 A & CN 109715788 A & JP 2019-519246 A claims, paragraphs [0002]-[0005], [0009], [0046]-[0067], [0086]-[0100], [0117], [0122], [0133]-[0135] | 1-22 |
| Y | WO 2018/009847 A1 (AMERICAN GENE TECHNOLOGIES INTERNATIONAL INC.) 11 January 2018, paragraph [0377] & US 2019/0201523 A1 & EP 3481435 A1 & JP 2019-525914 A paragraph [0194] | 13-14 |
| Y | WO 2017/203356 A1 (THE COUNCIL OF THE QUEENSLAND INSTITUTE OF MEDICAL RESEARCH) 30 November 2017, page 1, line 17 to page 4, line 7 & JP 2019-516751 A paragraphs [0003]-[0006] & EP 3465203 A1 & CN 109477830 A & KR 10-2019-0028662 A | 13-14 |
| Y | WO 2016/076415 A1 (KYOTO UNIVERSITY) 19 May 2016, claims, paragraphs [0002], [0014]-[0015] & US 2017/0326175 A1 paragraphs [0002], [0024]-[0025] & EP 3219791 A1 | 1-22 |
| Y | WO 2011/096482 A1 (THE UNIVERSITY OF TOKYO) 11 August 2011, claims, paragraphs [0024], [0042] & US 2013/0078226 A1 claims, paragraphs [0116], [0160] | 1-22 |
| Y | JP 2017-158559 A (THE UNIVERSITY OF TOKYO) 14 September 2017, claims, paragraphs [0035], [0072] & US 2013/0078226 A1 claims, paragraphs [0116], [0160] & WO 2013/176197 A1 & EP 2853590 A1 | 1-22 |
| P, X | WO 2019/070021 A1 (THYAS CO., LTD.) 11 April 2019, claims, paragraphs [0018]-[0022], examples (Family: none) | 1-22 |

Form PCT/ISA/210 (continuation of second sheet) (January 2015)

**EP 3 831 936 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017534261 A
- JP 2017530694 A
- JP 2018514204 A
- JP 2017158559 A

**Non-patent literature cited in the description**

- **NISHIMURA T et al.** Generation of rejuvenated antigen-specific T cells by reprogramming to pluripotency and re-differentiation. *Cell Stem Cell,* 03 January 2013, vol. 12 (1), 114-126
- **R. K. LINDEMANN et al.** *Mol. Cancer,* 2003, vol. 2, 20
- **TAKAYAMA N. et al.** *J Exp Med.,* 2010, 2817-2830
- **NIWA A et al.** *J Cell Physiol.,* November 2009, vol. 221 (2), 3677-77
- **NIWA A et al.** *PLoS One,* 2011, vol. 6 (7), e22261